# EUROPEAN PATENT APPLICATION

(11) **EP 1 798 614 A2**
(43) Date of publication of application: **20.06.2007**
(21) Application number: 06291941.0
(22) Date of filing: 15.12.2006
(51) Int. Cl.: G05B 19/042, A61M 39/00

(54) **Sterile tubing welder system**

(30) Priority: 15.12.2005 US 750511 P
(71) Applicant: Terumo Medical Corporation, Somerset, New Jersey 08873 (US)
(72) Inventor: Bollinger, Joseph H., West Grove, PA 19390 (US); Ahn, Janet J., Dumont, NJ 07628 (US); Reilly, Patricia A., Brooklyn, NY 11209 (US); Miripol, Jeffrey E., Hockessin, DE 19707 (US)
(74) Representative: Santarelli

(57) **Abstract**

A data management system for use with sterile tubing welders that documents welding process information and provides traceability of the individual welds made by the sterile tubing welder. The data management system includes a communication module, a tubing welder and application software, and documents information and provides traceability of each sterile connection made by the welder. The communication module creates an electronic record each time a weld is performed on the tubing welder. The application software creates connection process definitions for the communication module, uploads connection records from the communication module, and allows review of the connection records.

## Description

This invention claims the benefit of U.S. Provisional Application Ser. No. 60/750,511 filed on December 15, 2005.

### BACKGROUND OF THE INVENTION

### Field of the Invention

The present invention relates to a docketing system for the welding of sterile tubing, and more specifically, to a computerized docketing system for sterile tube welding made from sterile tubing welding apparatuses.

### Prior Art

The prior art provides several sterile tubing welding machines for sterilely connecting sterile, closed end tubes. The process used in conjunction with the welding machine comprises flattening a section of each tube to urge inside walls of each tube into contact, urging a hot cutting means through the flattened section of each tube thereby temporarily sealing together the inside walls of each tube and providing molten tube ends, aligning the tubes to be connected with each other, joining the desired molten ends of the tubes together to form a joint between the tubes, and cooling the joint and then subjecting it to stress to open the temporary seal in each tube, thereby providing fluid communication between the joint tubes.

Sterile tubing welding machines, such as the one disclosed in U.S. Patent No. 4,610,670, comprise a cutting means, means adapted to heat the cutting means, a pair of mounting blocks adapted to receive, hold and flatten the tubes to be joined, means to provide movement between the blocks and the cutting means to a position such that the cutting means is between the blocks and traversing where the blocks are adapted to hold tubes, means for realigning the blocks to a position where two different tube ends are aligned with and facing each other, means to separate the blocks and the cutting means, and means for urging the mounting blocks together. The tubes to be connected according to this process have closed ends, i.e., the tubes have sealed ends, a tube is connected to a container such as a blood bag or dialysis bag, the tube is connected to a catheter implanted in a patient, or in some other manner the tube ends are closed to the external environment. Other patents are disclosed in U.S. Pat. Nos. 4,369,779, 4,461,951, 4,476,631, and 4,501,951.

In the course of using the aforementioned sterile tubing welding machine, customers as well as the FDA have requested that the manufacturer provide documentation that satisfies certified good manufacturing practices and quality assurance requirements relating to manufacturing protocols applicable to the welding machine. To provide customers with statistical documentation that their welding machine is operating within the manufacturer's strength specifications and to provide verification that the customer is utilizing the welding machine properly, and to provide the FDA with proper documentation of their requirements, it would be beneficial to provide a method and apparatus of verifying the quality, number, location and other statistical data of welds and tubing made with the welding machine in the form of a convenient electronic system.

U.S. Patent No. 5,888,238 provides for a kit for testing the integrity of sterile tubing welds made by a sterile tubing welding machine employing welding wafers includes a plurality of tubing samples, and a container having a series of receptacles for holding the tubing samples and the wafers used in the sterile tubing welding machine. Further, a set of instructions is provided for making the sterile tubing welds with the tubing samples in the container, and a data sheet is used for recording particulars of the kit and reporting a weld analysis. The data sheet is generally completed after the welds are made. The data sheet generally includes information such as a customer label, certain particulars of the kit including the serial number of the welding machine, and analysis feedback to be sent back to the customer reporting the findings of the manufacturer having examined the welds made with the kit.

In data sheets used in the prior art, it is critical that certain information be input on the data sheet before certain steps are taken in the welding process, and more critical that these steps or information are documented. Due to human error, certain information may not be entered on a data sheet, certain steps in the process can be forgotten, skipped or overlooked, the data sheet can be lost or misplaced, incorrect information can be entered on parts of the data sheet, or the information can be incorrectly transcribed. Additionally, once the information has been documented, any person that views the data sheet may be able to enter or delete information, the information may be lost, or an unauthorized person may view confidential information.

What is needed in the art is an automated sterile tubing welding system by which the manufacturer may easily track the working progress of each welding tube and each welding machine so that individual customers can be informed of the quality of welds made by their machines, and be able to track the welding tubes made. There is a need in the art for an automated system to document welding process information and provide traceability of each sterile connection made by each welder. It is also necessary to provide a system that provides user access rights, processes specific access rights, creates and downloads reports, walks the user through each step of the sterile connecting process and creates sterile connecting processes. It would also be desirable to create a system and software which improves the communication between the manufacturer and the customer of the sterile tubing weld machine.

### SUMMARY OF THE INVENTION

The present invention satisfies the above-mentioned need by providing a data management system for use with sterile tubing welders that documents welding process information and provides traceability of the individual welds made by the sterile tubing welder. The data management system can include a communication module, such as a touch screen, a barcode reader and application software. The application software provides for specific user access rights, processes specific access rights, creates and downloads reports, and creates sterile connecting processes. The communication module can include documentation of the entire welding process, process control that provides for the completion of each step in order to proceed to the next step, visibility on the module of only trained/qualified processes for each user, and a bar code scan or manual entry of information.

Accordingly, a data management system is provided, comprising a tubing welder that performs welds, and a communication module in communication with the tubing welder for creating one or more connection record(s) when a weld is performed by the tubing welder. The data management system can further comprise application software for uploading the connection record(s) from the communication module, and a personal computer for storing the application software. The uploaded connection record(s) can be stored in the personal computer.

The application software creates connection process definitions for the communication module, and the communication module can be wired or wirelessly connected to the application software. The data management system can further comprise a status indicator on the communication module indicating the status of the connection between the communication module and the application software and/or tubing welder.

The application software can assign individual users to specific processes with varying security levels, and provides the ability to create and download reports of the connection record(s). The application software also provides the ability to create connection processes for the communication module, and the ability to assign specific data descriptors for each connection process. The application software can provide the ability to sort weld data by data descriptors, and indicate where the connection record(s) are being stored. The application software can also provide a status indicator indicating the status of the connection between the communication module and the application software.

The application software further provides a list of data descriptors that can be viewed per connection record uploaded from the communication module, which can be available filters or active filters. The application software provides a summary of the connection record(s), including date, time, identification of user and action taken, and allows for password changes, user configurations, process configurations, assignment of users to specific processes and different communication module configurations.

The data management system further comprises a scanner connected to the communication module for scanning information of items to be welded and storing the information in the communication module to be uploaded into the application software. The scanner can comprise a laser barcode scanner, or a manual entry keypad. The information of items can comprise barcoded information or manually entered text using a manual entry keypad on the communication module.

The communication module can be wirelessly connected to the tubing welder. The communication module documents the entire weld process performed by the tubing welder, and ensures each step in the weld process performed by the tubing welder is completed before proceeding to a next step. The communication module can discontinue the weld process of the tubing welder if any step is not correctly performed, and can provide a notification to a user if any step is not correctly performed or if the incorrect information has been scanned/entered.

The communication module can comprises a touch screen display. The communication module can further provide a user with step by step instruction on the use of the communication module and/or tubing welder, and can indicate a progress of a weld process performed by the tubing welder. The communication module also records whether each connection record(s) was a completed weld or not.

The communication module can display a message to a user and/or terminates a weld process of the tubing welder if the communication between the tubing welder and communication module is interrupted, delayed or disconnected, and further can provide instructions to the user on how to correct the communication between the tubing welder and the communication module. The communication module is where the user inspects each weld made by the tubing welder and electronically documents whether the weld was accepted or rejected by the user. These records whether the inspected weld has been accepted or rejected is stored on the communication module to be uploaded to the application software.

Further, a data management system is provided, comprising a sterile tubing welder for connection of one or more items, a scanner for recording a bar code provided on each item, a communication module for storing the information recorded by the scanner, the communication module being in communication with the sterile tubing welder for creating one or more connection record(s) when a weld is performed by the tubing welder, and application software for uploading the connection record(s) from the communication module. The communication module is where the user records the inspection of each weld and accepts or rejects the weld. Also, the communication module is where information for each item is recorded by the scanner.

Also provided is a method of data management, the method comprising performing a weld between one or more items, creating a connection record of each weld, and uploading and storing each connection record of each weld. Each weld information recorded can be permanent and cannot be altered. Preferably, only notes or comments can be made to each weld information and this information is traceable by name, date and time stamp. The method further comprises inspecting each weld and accepting or rejecting the weld, and storing this information. The method further comprises scanning information of each item so that the weld is performed, and documenting a weld process from start to finish of all items that are welded.

The above and other features of the invention, including various novel details of construction and combinations of parts, will now be more particularly described with reference to the accompanying drawings and pointed out in the claims. It will be understood that the particular device embodying the invention is shown by way of illustration only and not as a limitation of the invention. The principles and features of this invention may be employed in various and numerous embodiments without departing from the scope of the invention.

### BRIEF DESCRIPTION OF THE DRAWINGS

These and other features, aspects, and advantages of the apparatus and methods of the present invention will become better understood with regard to the following description and accompanying drawings where:
Figure 1 illustrate a schematic representation of the system of the present invention; and
Figure 2 illustrates a flow diagram of a sterile connecting process using the system of Figure 1.

### DETAILED DESCRIPTION OF THE INVENTION

The present invention provides for an accessory item that is intended for use with sterile tubing welders to document information and provide traceability of each sterile connection made by the welder. The system design and requirements provide comprehensive process control enabling the user to effectively manage the sterile connection process.

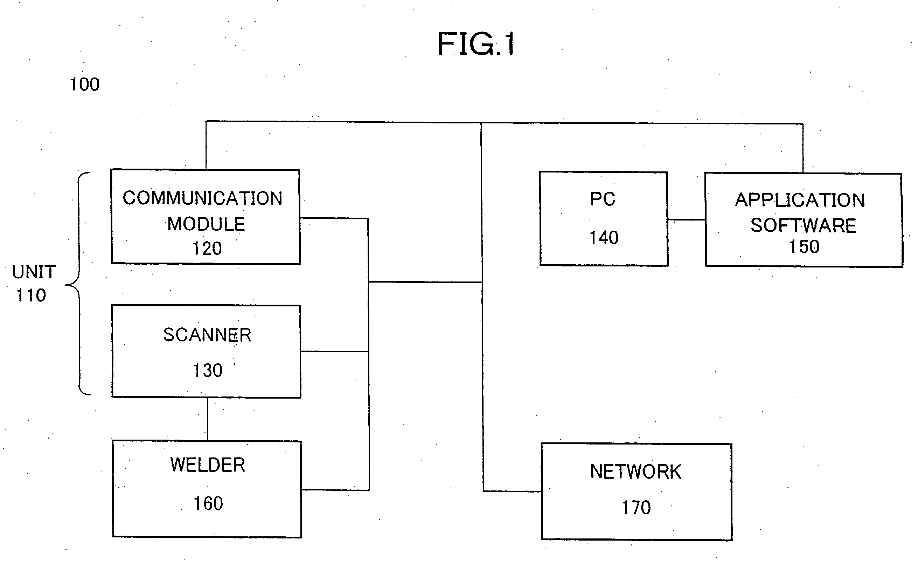

As shown in Fig. 1, a system 100 is provided that comprises a communication module 120, which can be a touch screen, a scanner 130, a personal computer (PC) 140, application software 150 for the PC, and/or any required cabling. Other components of the system 100 may comprise a tubing welder 160, and a network 170 that can be hard wired or wireless. The major components of the system 100 are the tubing welder 160, the communication module 120 and the application software 150.

The tubing welder 160 provides a sterile weld between two pieces of polyvinyl chloride (PVC) tubing in combinations of dry/dry, wet/dry and wet/wet tubing, but is not limited to the above. Tubing may be attached to such items as (but not limited to) bags, needles, filters, collection sets, infusion sets or apheresis sets, or any other items that would need to be welded as would be obvious to one of ordinary skill in the art. The sterile weld between two closed internally sterile components facilitates blood and component collection, processing and therapeutic procedures. Applications include, but are not limited to, blood and blood component collection, separation, filtration, sampling, treatment, additive introduction, pooling and such automated procedures as apheresis, cell washing and freezing.

The use of the tubing welder permits sterile connection in a variety of applications. Some of the more common uses of the Tubing Welder are to:
- Add a leukocyte reduction filter.
- Remove samples from blood product containers for testing.
- Add a new gauge needle to a blood collection or apheresis processing set.
- Prepare components: e.g. add one or more bags, as specifically needed, for component separation after collection of whole blood in a single bag; add one or more bags when additional components are needed, e.g. make a triple set out of a double.
- Pool blood products.
- Prepare an aliquot for pediatric use and divided units, e.g. attach a syringe set or a set of bags to prepare aliquots of whole blood, red blood cells or plasma.
- Connect additional saline or anticoagulant lines during an automated apheresis procedure.
- Connect a bag of processing or additive solution or reconnect one that was mistakenly removed.

There are many other instances where it is desirable to maintain a closed system at a connection site rather than to use the traditional "spiking" procedure.

The prime function of the communication module 120 is to create an electronic record each time a weld is performed on the tubing welder 160. The system 100 is capable of performing over a wired Ethernet interface or a wireless network. The major function of the application software 150 is to manage how each communication module 120 will operate, who can operate on it, and what will be allowed to be done on it. The application software 150 creates connection process definitions for the communication module 120, to upload the connection records from the communication module 120, and to allow supervisors to review the connection records. Connection process definitions are steps that specify data to be collected and actions to be performed when creating a connection. Connection records are data that is collected each time a connection is performed.

Each sterile tubing welder 160 can be connected to the communication module 120 and scanner 130 (by cable or wirelessly), which together can be considered a unit (workstation) 110. There can be from 1 to 256 units or more connected to one PC, and the application software 150 is installed on the PC. The sterile tubing welder 160 and barcode reader 130 are connected to the communication module 120, via cable or wirelessly. The communication module 120 can be hard-wired or wirelessly connected to the PC 140. Also, each unit 110 may be used on-line (connected with the PC 140) or off line (not connected to the PC 140). Wireless connections are contemplated in the invention as an antenna/receiver combination, or using Bluetooth® technology, or any other means known in the art.

When a unit is used off-line, it can store from 1 to 5,000 or more records of weld information. Preferably, once the storage of the unit has reached near the full capacity of welds, it will start alerting the individual user that it is approaching memory capacity and will require download of the information to a PC 140. Once each unit is then reconnected to a PC, the records or weld connection information on the system will be automatically downloaded and thus clearing memory from the communication module 120.

Each unit 110 can provide process controls by allowing the user to proceed only when the requested information has been provided. Essentially, each unit 110 will document information for each weld connection made by the associated sterile tubing welder 160.

The communication module 120 is capable of communication with the application software 150 and the sterile tubing welder via a hard wired interface or wirelessly. The communication module 120 also provides a status indicator that indicates the status of the communication links with the application software 150 and/or the sterile tubing welder 160. The communication module 120 can also control when the sterile tubing welder 160 performs a weld. The application software 150 uploads connection records and connection processes from the communication module 120. The communication module 120 is fully functional when it is online (connected to the application software 150) or offline.

The communication module 120 is capable of reading any type of barcode labels from the scanner 130, and supports a laser or any other type of scanner. The scanner is not limited to a barcode scanner, but can also be a manual input means, or any other type of data entry means that allows for the communication module 120 to read the information of each item for the sterile connecting process. The application software 150 allows a supervisor to configure barcode matching rules including start and end character match and barcode length. The communication module 120 provides the capability for the user to display the contents of a scanner bar code label when a connection process is not being executed.

The communication module 120 features provide but are not limited to the following:
- Ability to electronically document the entire welding process from start to finish by scanning a barcode or manually entering in text.
- Ensure process control in which each step within a process must be completed in order to proceed to the next.
- Ensure each step or scanned information matches with any applicable barcode matching rules.
- Additional control provided by visibility of only trained/qualified processes for each user.
- Ensures system and process time outs as part of the security features.
- Ability to select processes at the workstation.
- Ability to make comments during weld inspection (by scanning a bar code or manually entering the information).
- Capable of storing over 5,000 or more connection records without the need for downloading connection records to the PC. This amount can be increased with increased memory space, as would be obvious to one or ordinary skill in the art.
- Supports the recording of a minimum of 350 welds per 8 hours.

The communication module 120 can house a touch screen display. The touch screen on the communication module 120 can allow the user to enter information as specified by the processes created on the application software 150. Information can be entered by bar code scanning or by manual entry methods or any other data entry method as would be obvious to one of ordinary skill in the art.

The communication module 120 can have a touch screen that uses both symbols (icons) and words to instruct the user on the use of the instrument. The screens can be divided into four main areas. These areas include the title bar, the current step area, the process information area and the progress bar and control button area.

The title bar can include information such as the process title/name, the date and time, and user identification. The title bar can also include a menu button to select one or more of the following options: it can scan a barcode to reveal hidden characters by reading a barcode (accessible by all users), a logout button (accessible by all users), and an exit button to setup (accessible by administrator only).

The current step area can show the current step being performed within the process. The process information area can include a keypad for manual entry, can show the previous step entered, can show instructions on the current step, and show the next step as well.

The progress bar and control button area can show the percentage of progress within the specific sterile connecting process, and can have a terminate process button and allow for return to the selected process.

The communication module 120 supports the following connection process steps: Operator identification, sterile tubing welder identification, wafer cassette identification, bar code scans, connection inspections, comment entry or barcode scan upon inspection, and performs connections with a date and time stamp, if desired. The application software 150 can configure the communication module 120 to timeout during a connection process if an operator interaction has not occurred within a configured time. The communication module 120 can also account for wafers which have been removed from their packaging, but not yet used in the formation of a connection. The communication module 120 also guides the user through the connection process.

Generally, the communication module 120 connection process collects all data and inputs, performs the connection, allows inspection of the connection with acknowledgement from the operator and logs the connection record.

The communication module 120 maintains connection processes in a non-volatile memory so that the processes are not lost after a power cycle. The communication module 120 performs a quality control check (a weld that is performed on a periodic basis to confirm the operation of the sterile tubing welder 160 and/or with every new wafer lot number) when configured to do so by the application software 150. During steps in the weld process, the communication module 120 can display custom text that has been previously defined by the application software user. If any error is detected during the weld process by the communication module 120, then the weld process will not continue and no connection will be made. Also, the weld process can be terminated, with no weld being performed. Such information will be recorded by the communication module, and it will record all information scanned or manually entered and note that the weld was unsuccessful as the information is previously scanned before the weld process is performed.

If the communication between the sterile tubing welder 160 and the communication module 120 is interrupted, delayed or disconnected, a message can be displayed to the operator indicating that a communication failure has taken place between the communication module 120 and the welder, and the weld process can be terminated before the weld takes place. If the weld has occurred and the communication failure occurs, the communication module 120 can indicate that a communication error has occurred to the operator. If the weld is occurring and the communication failure occurs, the communication module 120 can indicate that a weld error has occurred to the operator, and the weld can be discontinued. The communication module 120 can guide the user as to what steps are to be taken to correct the situation.

The application software 150 can be a program that resides on a computer (PC) 140. The software documents information scanned or manually entered in the communication module 120 in use with the sterile tubing welder 160. The application software 150 can include a status indicator indicating the status of the connection between the communication module 120 and the application software 150.

The application software 150 supports the following activities: software installation, software upgrade, password configuration, user configuration, process configuration (definition and installation), communication module configuration, sorting/filtering of weld information and connection report generation, viewing and printing. The application software 150 allows the communication module 120 to be configured to read and remember information that is repeatedly gathered during a weld process, and add the remembered information to the weld record without user intervention during a specified time period or session. The application software 150 and the communication module 120 support any connection process created by the user where multiple bags are welded together serially on one communication module 120. The application software 150 also transfers connection processes to and from the communication module 120.

The application software 150 features provide but are not limited to the following:
- Information captured is pre-determined during setup for each process and includes, but is not limited to, lot numbers, product code, and user identification.
- Assign individual users to specific processes with varying security levels.
- Ability to create and download reports.
- Ability to assign specific data descriptions for each process created.
- Ability to sort weld data by data descriptions (column headers).
- Assign individual users access rights as determined by the administrator. (Levels include administrator, operator, and supervisor.)
- Ability to assign the associated sterile tubing welder to be disabled from use as part of the unit 110.
- Ability to configure and store in memory up to 64 or more different sterile connecting processes.
- Provides data integrity though encryption.
- Stores connection records in files as specified by the user (i.e., separated by and identifiable by the day or hour they are performed.
- Allows multiple users to access connection record fields.
- Capable of uploading connection records to a facility's main data storage location.
- Allows a qualified user to indicate that a connection record has been reviewed, and attach an electronic comment to a connection record.
- Capable of displaying the IP address, software version, serial number and the name of the communication module 120 that created the connection record.
- Provides the ability for users to view or generate reports that contain information from connection records.
- Ability to assign system weld acceptance and process timing out for each communication module 120.
- Ability to require a password entry at weld acceptance and at log out for each communication module 120.
- Supports more than 1,000 active and inactive communication module and application software users. This amount can be increased with increase memory space, as would be obvious to one of ordinary skill in the art.

The application software screen can use shortcut keys and pull-down menus to allow the user to program the information to the communication module 120. The screen can be divided into five main areas: a status area, a menu area, a shortcut key area, a communication module listing area and a display area.

The status area can indicate the directory file where sterile connection records (data collected each time a connection is performed) are being stored, the display file that is currently being viewed in the display area, the status of communication with the communication module, if the file is available to add comments and/or review and create reports, and can also indicate the status where the file is able to be updated with additional sterile connection records.

The menu area can have an application configuration that is not accessible by operator level users. It can further have a site name that indicates the facility name which is displayed as the location or site name when creating reports in excel or html. The menu area can have a default directory that sets the path and allows sterile connections to be stored to a specified file using a browse button. The menu area can have a communication module network discovery polling that sets the time intervals (in minutes or seconds) for the Application Software 150 to search and identify active communication module(s). The menu area can further have a setting for retrieving connection record that sets the time interval (in minutes or seconds) for the Application Software 150 to search and upload sterile connection records from active Communication Module(s).

The menu area can further have a 24-hour clock synchronization setting that sets the time when the clock on the Application Software 150 will synchronize with active Communication Module(s). The menu area can further have a setting enabling review of connection records that enables review of each sterile connection record. The information in the menu area is preferably accessible by a user with administrator level access rights.

The menu area further has a communication module license file setting, which allows the user to select a license file. Selecting the correct license file ensures that the application software will recognize the specific communication module. The menu area can further have a setting to log out, and close the application software. The menu area further has a password duration setting, which allows the user to identify the length of time for the expiration of the current password(s).

The menu are further has a view setting, which allows for a view of displayed columns. The user can choose to view available columns also, which is a list of data descriptors that can be viewed per sterile connection record uploaded from the communication module(s). The user can move one item off the list of available columns to a list of displayed columns. The displayed columns list is a list of data descriptors that is currently in view for each sterile connection record uploaded from the communication module(s). The user can customize the information to be collected per sterile connection record. Data descriptors can be moved from the available columns list to the displayed columns list, and vice versa, and can be added and removed as well.

A display configurations setting allows the user to save or delete any display configuration changes made above. An add columns setting from a connection record feature allows the user to add any and all columns for any connection record(s) being viewed in the display area.

A connection history setting allows the user to highlight a specific sterile connection record first from the display area and select this feature to display a quick summary of the specific record including date, time, identification of user and action taken (i.e. marked, reviewed, etc.).

The menu area further has a setting for connection record filters, which can be available filters or active filters.

Available filters are a list of data descriptors that can be used to filter the sterile connection records uploaded from the Communication Module(s). Using a list of maneuvering buttons, the user can move items off this list to the Active Filters list. These data descriptors can include an accept/reject setting to view accepted or rejected welds, a communication module serial number setting to display or not display the appropriate serial numbers, a custom search setting (identifying any of the data descriptors specified for each sterile connecting process), a date of weld setting to identify a specific date, a range in date, a before or after date, a reviewed/not reviewed setting to view reviewed or non-reviewed records, incomplete welds, operator name, records containing manual entries, comments, reviewer, date of review and a welder serial number setting to see if the records contain the appropriate device serial number.

The active filters are a list of data descriptors that can be used to filter the sterile connection records uploaded from the Communication Module(s). Using a list of maneuvering buttons, the user can move one item off this list to the Available Filters list.

The menu are further includes settings such as record storage information setting for viewing a record file that indicates the current file visible in the Display Area, and a storage record file that indicates the file where sterile connection records are being stored. The menu area further includes settings such as a network filter, a search filter to identify connection with active Communication Module(s), a remove unit setting to manually refresh the list of active Communication Module, a remove all units setting to manually refresh the list of active Communication Module(s) (this selection would eliminate all active Communication Module only in the Communication Module Listing Area), an update unit clock setting to synchronize the clock on an active Communication Module available in the Communication Module Listing Area, and an update all units clocks setting that synchronizes the clocks on all active Communication Module(s) available in the Communication Module Listing Area.

The menu area further has a configuration setting that allows for password changes, user configurations, process configurations, that assigns users to processes and provides for different communication module configurations.

The application software 150 can also have a shortcut key area that provides for various shortcuts for opening and retrieving files and/or records, reviewing records, searching for networks, adding comments and setting filters. The application software 150 further has a communication module listing area that provides a list of all communication modules currently connected, and a display area that provides a list of data descriptors currently in view for each sterile connection record. This information can be customized.

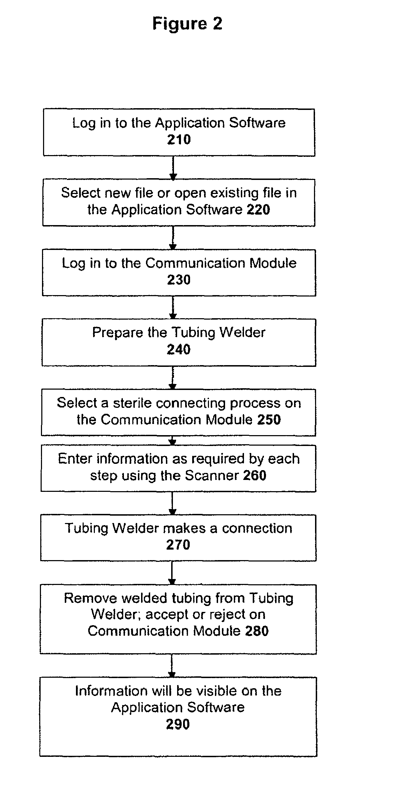

Figure 2 shows a flow diagram of a sterile connecting process using the system 100. First, a user can log in to the application software at step 210. To log in, the user must enter a usemame and enter a password at step 210. Then, the user must select a new file or open an existing file at step 220. The user then logs in to the communication module at step 230. To log in, the user must enter a user name and/or enter a password at step 230. The sterile tubing welder 160 is then prepared at step 240, and the tubing placement can be confirmed. The user then selects a sterile connecting process at step 250. Preferably, before the process starts, the user has an option to select a different connecting process, which can all be listed on one page or multiple pages.

At step 260, information is scanned as required by each step using the scanner 130. Each barcode can be scanned using the scanner 130, which is a tool that can be used to read hidden characters in a particular bar code. Data entry may be entered by scanning a bar code or using a keypad for manual entry. When scanning a bar code, the scanner 130 connected to the communication module 120 is used. Preferably, only the information requested at each step is scanned. A keypad for manual entry may also be used, which allows data to be entered manually rather than by bar code scanning. When a button on the keypad is touched, a manual entry screen can be displayed. Depending on the specific step within the process, different manual input screens may appear. All data that cannot be scanned in using the barcode scanner 130 can be entered manually. With all manually entered information, the system can request a second entry and/or a confirmation screen can appear with the manually entered information to ensure the correct information is entered.

At step 270, the sterile tubing welder 160 makes a connection. At step 280, the welded tubing is removed from the welder 160, the tubing is inspected and the weld is either accepted or rejected. The user inspects the weld made by the tubing welder and electronically documents on the communication module whether the weld was accepted or rejected. This information is recorded whether the particular weld was rejected or accepted, and saved based on the information scanned in by the scanner 130, and later uploaded into the application software. The weld information recorded is preferably permanent and cannot be altered. Only notes or comments can be made to the weld information and this information is traceable by but not limited to name, date, time stamp.

The user can then stay logged in or choose to logout of the communication module. In either case, the weld information will appear on the application software at step 290.

The method and system of the present invention provides several advantages that solve the problems with the prior art. The present invention provides an automated docketing of the welding process, and records information on each weld performed and any products (e.g., bags) or information (e.g., wafer lot number) that are involved in the welding process. The present invention provides a data management system for use with sterile tubing welders that documents welding process information and provides traceability of the individual welds made by the sterile tubing welder. The application software provides for specific user access rights, processes specific access rights, creates and downloads reports, and creates sterile connecting processes. The communication module includes documentation of the entire welding process, process control that provides for the completion of each step in order to proceed to the next step, visibility on the module of only trained/qualified processes for each user, and a bar code scan or manual entry of information. The present invention provides for an accessory item used with sterile tubing welders to document information and provide traceability of each sterile connection made by the welder. The system design and requirements provide comprehensive process control enabling the user to effectively manage the sterile connection process.

The above descriptions of the present invention are specific embodiments of the present invention and are not limited to the above descriptions and uses. While there has been shown and described what is considered to be preferred embodiments of the invention, it will, of course, be understood that various modifications and changes in form or detail could readily be made without departing from the spirit of the invention. It is therefore intended that the invention be not limited to the exact forms described and illustrated, but should be constructed to cover all modifications that may fall within the scope of the appended claims.

## Claims

1. A data management system comprising:
a tubing welder that performs welds; and
a communication module in communication with the tubing welder for creating one or more connection record(s) when a weld is performed by the tubing welder.

2. The data management system of claim 1, further comprising:
application software for uploading the connection record(s) from the communication module.

3. The data management system of claim 2, further comprising:
a personal computer for storing the application software.

4. The data management system of claim 3, wherein the uploaded connection record(s) are stored in the personal computer.

5. The data management system of claim 2, wherein the application software creates connection process definitions for the communication module.

6. The data management system of claim 2, wherein the communication module is wirelessly connected to the application software.

7. The data management system of claim 2, further comprising:
a status indicator on the communication module indicating the status of the connection between the communication module and the application software and/or tubing welder.

8. The data management system of claim 2, further comprising:
a status indicator on the application software indicating the status of the connection between the communication module and the application software.

9. The data management system of claim 2, wherein the application software assigns individual users to specific processes with varying security levels.

10. The data management system of claim 2, wherein the application software provides the ability to create and download reports of the connection record(s).

11. The data management system of claim 2, wherein the application software provides connection processes for the communication module.

12. The data management system of claim 11, wherein the application software provides the ability to assign specific data descriptors for each connection process.

13. The data management system of claim 2, wherein the application software provides the ability to sort weld data by data descriptors.

14. The data management system of claim 2, wherein the application software indicates where the connection record(s) are being stored.

15. The data management system of claim 2, wherein the application software provides a list of data descriptors that can be viewed per connection record uploaded from the communication module.

16. The data management system of claim 15, wherein the data descriptors are available filters.

17. The data management system of claim 15, wherein the data descriptors are active filters.

18. The data management system of claim 2, wherein the application software provides a summary of the connection record(s), including date, time, identification of user and action taken.

19. The data management system of claim 2, wherein the application software allows for password changes, user configurations, process configurations, assignment of users to specific processes and different communication module configurations.

20. The data management system of claim 1, further comprising:
a scanner connected to the communication module for scanning information of items to be welded and storing the information in the communication module.

21. The data management system of claim 20, wherein the scanner comprises a laser barcode scanner.

22. The data management system of claim 20, wherein the scanner comprises a manual entry keypad.

23. The data management system of claim 1, wherein the communication module is wirelessly connected to the tubing welder.

24. The data management system of claim 1, wherein the communication module documents the entire weld process performed by the tubing welder.

25. The data management system of claim 24, wherein the communication module ensures each step in the weld process performed by the tubing welder is completed before proceeding to a next step.

26. The data management system of claim 25, wherein the communication module discontinues the weld process of the tubing welder if any step is not correctly performed.

27. The data management system of claim 25, wherein the communication module provides a notification to a user if any step is not correctly performed.

28. The data management system of claim 1, wherein the communication module comprises a touch screen display.

29. The data management system of claim 1, wherein the communication module provides a user with step by step instruction on the use of the communication module and/or tubing welder.

30. The data management system of claim 1, wherein the communication module indicates a progress of a weld process performed by the tubing welder.

31. The data management system of claim 1, wherein the communication module records whether each connection record(s) was a successful weld or not.

32. The data management system of claim 1, wherein the communication module displays a message to a user and/or terminates a weld process of the tubing welder if the communication between the tubing welder and communication module is interrupted, delayed or disconnected.

33. The data management system of claim 33, wherein the communication module provides instructions to the user on how to correct the communication between the tubing welder and the communication module and if necessary, continue the unfinished welding process.

34. The data management system of claim 1, wherein the communication module records whether each weld inspected by the user was accepted or rejected.

35. The data management system of claim 34, wherein the communication module records whether the inspected weld has been accepted or rejected.

36. A data management system, comprising:
a sterile tubing welder for connection of one or more items;
a scanner for recording a bar code provided on each item;
a communication module for storing the information recorded by the scanner, the communication module being in communication with the sterile tubing welder for creating one or more connection record(s) when a weld is performed by the tubing welder; and
application software for uploading the connection record(s) from the communication module.

37. The data management system of claim 36, wherein the communication module further records whether each weld was accepted or rejected by a user, and stores this information for each item recorded by the scanner.

38. A method of data management, the method comprising:
performing a weld between one or more items;
creating a connection record of each weld; and
uploading and storing each connection record of each weld.

39. The method of data management of claim 38, the method further comprising:
inspecting each weld and accepting or rejecting the weld, and storing this information.

40. The method of data management of claim 38, the method further comprising:
scanning information of each item so that the weld is performed.

41. The method of data management of claim 38, the method further comprising:
documenting a weld process from start to finish of all items that are welded.
